# EUROPÄISCHE PATENTANMELDUNG

(11) **EP 0 834 546 A2**
(43) Veröffentlichungstag der Anmeldung: **08.04.1998**
(21) Anmeldenummer: 97116515.4
(22) Anmeldetag: 23.09.1997
(51) Int. Cl.: C11D 1/12, C07C 303/32

(54) **Tensidmischungen mit einem Gehalt an Acyloxialkansulfonaten**

(30) Priorität: 01.10.1996 DE 19640571
(71) Anmelder: Clariant GmbH, 65929 Frankfurt am Main (DE)
(72) Erfinder: Nestler, Bernd, Dr., 65929 Frankfurt (DE)

(57) **Zusammenfassung**

Tensidmischungen mit einem Gehalt an Acyloxialkansulfonaten, hergestellt durch Umsetzung einer oder mehrerer Fettsäuren mit (a) einem Alkali- und/oder Erdalkalihydroxialkansulfonat oder (b) einem Gemisch aus Alkali- und/oder Erdalkalihydroxialkansulfonat und Hydroxialkansulfonsäure, wobei vor, während oder nach Beendigung der Reaktion im Fall (a) ein Ammoniumsalz und im Fall (b) ein Ammoniumsalz oder ein Amin zugegeben wird.

## Beschreibung

Acyloxialkansulfonate stellen anionische Tenside dar, die als Rohstoffe für Syndet-Seifen, kosmetische Mittel und Reinigungsformulierungen Verwendung finden. Sie zeichnen sich durch gute Schaumeigenschaffen, gute Hartwasserstabilität und gute Hautverträglichkeit aus.

Nachteilig für die Verwendung dieser Tenside ist, daß es sich dabei meist um spröde Feststoffe handelt, die erst bei hohen Temperaturen schmelzen bzw. rührbar sind. Bei diesen hohen Temperaturen, die erforderlich sind, um das Acyloxialkansulfonat überhaupt erst verarbeitbar machen, ist dieses sehr oxidationsempfindlich, eine thermische Zersetzung setzt ein und Verfärbungen treten auf.

Demnach ist es vorteilhaft den Schmelzpunkt bzw. die Temperatur, bei der Acyloxialkansulfonate rührbar und somit verarbeitbar sind, zu senken. Zur Lösung dieses Problems ist es bereits bekannt, solche Acyloxialkansulfonate herzustellen, die gemischte Salze darstellen, wo also das Kation aus einem Gemisch von zwei unterschiedlichen Kationen, beispielsweise dem Natrium- und dem Kalium-Ion besteht (US 3 029 264). Diese Acyloxialkansulfonate, wobei es sich vorzugsweise um Acylisethionate handelt, werden durch Veresterung von Fettsäuren mit einem Salz der Isethionsäure hergestellt. Nimmt man nun entsprechend den Angaben des genannten Standes der Technik ein Gemisch von Natriumisethionat und Kaliumisethionat, so kann man die Veresterung mit der Fettsäure bereits bei 150 bis 160°C durchführen. Auf diese Weise läßt sich die Bildung von Verfärbungen vermeiden. Bei diesem Verfahren werden jedoch in allen Fällen äquimolare Mengen an Fettsäure und Isethionat umgesetzt.

Acyloxialkansulfonate mit gemischten Kationen sind auch in WO 94/09107 beschrieben. Als Kationen kommen dort jedoch nur Natrium-, Kalium- und Magnesium-Ionen in Frage.

Gegenstand der Erfindung sind Tensidmischungen mit einem Gehalt an Acyloxialkansulfonaten, hergestellt durch Umsetzung einer oder mehrerer Fettsäuren mit (a) einem Alkali- und/oder Erdalkali-hydroxialkansulfonat oder (b) einem Gemisch aus Alkali- und/oder Erdalkali-hydroxialkansulfonat und Hydroxialkansulfonsäure, wobei vor, während oder nach Beendigung der Reaktion im Fall (a) ein Ammoniumsalz und im Fall (b) ein Ammoniumsalz oder ein Amin zugegeben wird.

Als Fettsäuren kommen gesättigte oder ungesättigte Fettsäuren in Frage mit einem Gehalt von 8 bis 32 C-Atomen. Als Beispiele seien genannt Capronsäure, Caprinsäure, Laurinsäure, Myristinsäure, Stearinsäure, Arachinsäure, Ölsäure, Linolsäure, Linolensäure. Bevorzugt sind Mischungen von Fettsäuren, wie beispielsweise Cocosfettsäure und Talgfettsäure. Neben unverzweigten sind auch verzweigte Fettsäuren geeignet, beispielsweise 2-Ethylhexansäure, 2-Pentyloctansäure, 2-Butylnonansäure, 2-Propyldecansäure, 2-Ethylundecansäure, 2-Butylundecansäure, 2-Methyldodecansäure 2-Ethyltridecansäure und 2-Methyltetradecansäure und Mischungen davon.

Die Hydroxyalkansulfonate entsprechen der Formel HO-R¹-SO₃X, worin R¹ -CH₂CH₂-, -(CH₂)₃-, -CH₂CH(CH₃)- oder -CH₂CH₂OCH₂CH₂- ist, wobei Ethylen bevorzugt ist.
X bedeutet hierin ein Alkali- und/oder Erdalkalikation, vorzugsweise Natrium, Kalium. Die hierzu korrespondierende Hydroxialkansulfonsäure hat die Formel HO-R¹SO₃H, wobei R¹ die zuvor angegebene Bedeutung hat.

Es ist von wesentlicher Bedeutung für die erfindungsgemäßen Tensidmischungen, daß die darin enthaltenen Acyloxialkansulfonate nicht ein einziges Kation, sondern ein Gemisch verschiedener Kationen haben, wobei mindestens eines dieser Kationen ein Ammoniumkation ist und der restliche Anteil Alkali- und/oder Erdalkalikationen ausmacht. Bevorzugt sind erfindungsgemäße Tensidmischungen, die so hergestellt werden, daß darin die Acyloxialkansulfonate mit folgenden molaren Verhältnissen den Kationen vorliegen: K:NH₄ von 97:3 bis 5:95; Na:NH₄ von 98:2 bis 5:95; vorzugsweise 97:3 bis 50:50; Na:NH(C₂H₅)₃ von 98:2 bis 10:90; K:NH(C₂H₅)₃ von 98,2 bis 10:90, Acyloxialkansulfonate mit gemischten Kationen und somit auch die solche Acyloxialkansulfonate enthaltenden erfindungsgemäßen Tensidmischungen lassen sich auf folgenden Wegen herstellen.

Zum einen kann man die Fettsäure oder Mischungen von Fettsäuren zunächst mit einem oder mehreren Alkali- und/oder Erdalkalihydroxialkansulfonaten umsetzen und dann nach Beendigung der Reaktion ein oder mehrere Ammoniumsalze zugeben.

Die Umsetzung der Fettsäure mit dem Hydroxialkansulfonat erfolgt nach an sich bekannten Verfahren, vorzugsweise nach dem Verfahren der sogenannten Direktveresterung durch Reaktion eines Überschusses von Fettsäure mit dem Hydroxialkansulfonat in Gegenwart eines Veresterungskatalysators bei einer Temperatur von 100 bis 260°C unter gleichzeitiger Entfernung von vorhandenem Wasser. Diese Direktveresterung erfolgt im einzelnen analog zu den Angaben in EP-A-0 585 071 (US 5,384,421), auf die hier ausdrücklich Bezug genommen wird.

Die Hydroxialkansulfonate können als solche eingesetzt werden, bevorzugt werden sie in Form einer wäßrigen Lösung, im allgemeinen als 40 bis 65 gew.-%ige Lösung, eingesetzt.

Man kann die Reaktion nur mit einem einzigen Hydroxialkansulfonat durchführen, beispielsweise mit Natrium-hydroxialkansulfonat, es ist aber auch möglich, Mischungen verschiedener Hydroxialkansulfonate zu nehmen, beispielsweise eine Mischung aus Natrium- und Kalium-hydroxialkansulfonat. Darüberhinaus kann man bereits an dieser Stelle einen Teil der benötigten Ammoniumkationen einfügen, indem man beispielsweise mit einem Gemisch aus Natrium- und/oder Kalium- sowie Ammoniumhydroxialkansulfonat arbeitet. Der dann noch fehlende restliche Anteil der insgesamt benötigten Menge an Ammoniumionen sind dann in Form eines Ammoniumsalzes wie weiter unten beschrieben zugegeben.

Geeignete Veresterungskatalysatoren sind in der genannten EP-A-0 585 071 ausführlich beschrieben, die hier miteinbezogen wird. Es handelt sich um Alkansulfonsäuren, Hydroxialkansulfonsäuren, Arylsulfonsäuren, anorganische Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, bezogen auf Hydroxyalkansulfonat.

Die Veresterung kann im einzelnen in der Weise durchgeführt werden, daß man bei Atmosphärendruck die Fettsäure, das Hydroxyalkansulfonat und den Veresterungskatalysator in einem Reaktionsgefäß vorlegt und die Mischung unter Rühren auf die oben angegebene Temperatur erhitzt. Das gegebenenfalls mit den Ausgangskomponenten in die Reaktionsmischung eingebrachte Wasser und das durch die Veresterungsreaktion entstehende Wasser wird dabei kontinuierlich aus der Reaktionsmischung ausgetragen. Darüberhinaus kann es auch sinnvoll sein, im Verlauf der Veresterungsreaktion oder im Anschluß daran einen Teil der überschüssigen Fettsäure abzudestillieren.

Man kann die Veresterungsreaktion auch anfangs bei Atmosphärendruck und dann unter Anwendung eines Vakuums zur schnelleren Austragung des Wassers durchführen. Im allgemeinen wird man, zum Beispiel aus Zeitgründen, einen 100%igen Umsatz nicht anstreben, sondern bei einem niedrigeren Prozentsatz, zum Beispiel bei 75 bis 90 Gew.-% Acyloxyalkansulfonat, die Veresterungsreaktion abbrechen, zum Beispiel durch Abkühlen. Dementsprechend liegt die Reaktionszeit im allgemeinen bei 4 bis 8 Stunden. Das erhaltene Reaktionsprodukt ist bei Raumtemperatur flüssig oder fest.

Während der Reaktion oder nach Beendigung der Reaktion, jedoch in jedem Fall vor oder während dem Abkühlen des Reaktionsgemisches wird ein Ammoniumsalz zur Reaktionsmischung zugegeben, um das andere Kation einzuführen.

Als Ammoniumsalze kommen Verbindungen der Formel

R¹, R², R³, R⁴ N^{⊕}X^{⊖}

in Frage, worin R¹, R², R³ und R⁴ gleich oder verschieden sein können und Wasserstoff, C₁-C₄-Alkyl oder C₁-C₄-Hydroxyalkyl und X ein Anion wie beispielsweise Chlorid, Sulfat oder das Anion einer C₁-C₂₀-, vorzugsweise C₁₂-C₁₈-Carbonsäure bedeuten. Beispiele für solche Ammoniumsalze sind Ammoniumchlorid und Ammoniumstearat. Auch bei den Ammoniumsalzen ist es möglich, Mischungen verschiedener Ammoniumsalze zu nehmen. Zusätzlich zu dem Ammoniumsalz kann man auch noch ein entsprechendes Alkali- und/oder Erdalkalisalz zugeben, beispielsweise als Mischung aus Natriumstearat und Ammoniumstearat. Die Menge an Ammoniumsalz beträgt 1 bis 95 mol%, bezogen auf die gesamte Menge an Acyloxialkansulfonat.

Durch die Zugabe dieser Ammoniumsalze erfolgt ein teilweiser Austausch der Alkali- und/oder Erdalkalikationen in dem Acyloxialkansulfonat gegen Ammoniumionen und man erhält die erfindungsgemäße Tensidmischung, die im wesentlichen ein Acyloxialkansulfonat enthält, dessen Kation ein Gemisch von Alkali- und/oder Erdalkalikationen und Ammoniumionen ist.

Bei der zweiten Variante zur Herstellung von Acyloxialkansulfonaten mit gemischten Kationen arbeitet man ebenfalls bevorzugt nach dem oben beschriebenen Verfahren der Direktveresterung von Fettsäure und Hydroxialkansulfonat, wobei hier ein Teil des Alkali- und/oder Erdalkali-hydroxialkansulfonats durch die korrespondierende freie Hydroxialkansulfonsäure ersetzt wird. Vorzugsweise beträgt der Anteil der freien Hydroxialkansulfonsäure 0,5 bis 50 mol.-% der Gesamtmenge an Hydroxialkansulfonat-Anion. Noch während oder auch nach Beendigung der Reaktion, d.h. vor oder während des Abkühlens des Reaktionsgemisches wird die freie Hydroxialkansulfonsäure durch Zugabe eines Ammoniumsalzes oder eines Amins neutralisiert. Als Ammoniumsalz können hierbei die oben definierten Ammoniumsalze genommen werden. Als Amine kommen Amine der Formel

R¹,R²,R³N

in Frage, worin R¹ bis R³ Wasserstoff, C₁-C₄-Alkyl, vorzugsweise Methyl oder Ethyl oder C₂-C₄-Hydroxiethyl bedeuten, wobei R¹ bis R³ gleich oder verschieden sein können.

Auch bei dieser Variante ist es möglich Acyloxialkansulfonate mit mehreren Arten von Kationen zu erzeugen, beispielsweise in der Weise, daß das Alkali- und/oder Erdalkali-hydroxialkansulfonat ein Gemisch aus Natrium- und Kalium-hydroxialkansulfonat ist, oder daß man zur Neutralisation ein Gemisch verschiedener Ammoniumsalze und/oder Amine nimmt.

Um die Viskosität des Reaktionsgemisches zu vermindern, kann man dem Reaktionsgemisch in allen Fällen vor oder während des Abkühlvorgangs sogenannte Konsistenzregler zugeben. Hierfür kommen in Frage beispielsweise Paraffine, wie in EP-A-0 585 071 beschrieben, Fettsäuren, Fettsäureester oder Polyethylenglykole oder Mischungen von Konsistenzreglern. Bevorzugt sind freie Fettsäuren, und zwar solche, die eine andere Kettenlänge haben als die zur Herstellung des Acyloxialkansulfonats benutzte Fettsäure. Der Anteil dieser Konsistenzregler kann bis zu 60 Gew.-%, vorzugsweise bis zu 30 Gew.-% betragen. Bevorzugt sind Mischungen mit bis zu
30 Gew.-% Paraffin, bis zu 50 Gew.-% Fettsäure und bis zu 10 Gew.-% Polyethylenglykol. Die Prozentangaben beziehen sich jeweils auf den Gehalt an das das Acyloxialkansulfonat enthaltende Material.

Die auf die beschriebene Art und Weise hergestellten Tensidmischungen, die als Hauptkomponente Acyloxialkansulfonate mit gemischten Kationen und daneben Restmengen an freien Fettsäuren und gegebenenfalls Konsistenzregler enthalten, zeichnen sich gegenüber ähnlichen Produkten nach dem Stand der Technik (US 3,029,264) dadurch aus, daß bei gleichem Gehalt an Acyloxialkansulfonat die Temperaturgrenze, bei der das Tensidgemisch noch rührbar ist, deutlich niedriger ist. Diese Absenkung der Rührbarkeitsgrenze bei den erfindungsgemäßen Tensidmischungen hat zur Folge, daß hier der Gehalt an Acyloxialkansulfonat höher sein kann als bei den Mischungen des genannten Standes der Technik. Eine weitere Absenkung der Rührbarkeitsgrenze kann durch Zugabe von Konsistenzreglern erfolgen. Darüberhinaus ist bei den erfindungsgemäßen Tensidmischungen der Restgehalt an freiem Isethionat deutlich niedriger.

### Beispiel 1

In einem 2 l Planschliffbecher mit Rührer, absteigender Destillationsbrücke, Innenthermometer und Stickstoffeinleitung wurden 231 g Cocosfettsäure, 255 g wäßrige Natriumisethionat-Lösung (58 %) und 0,7 g Zinkoxid vorgelegt. Der Ansatz wurde bei 210°C unter Abdestillieren des bei der Reaktion gebildeten Wasser bis zu einem Gehalt an waschaktiver Substanz von 75 % kondensiert und die derart erhaltene Schmelze mit 32,8 g Ammoniumstearat versetzt. Das so erhaltene Natrium-/Ammoniumcocoylisethionat-Gemisch war noch bei einer Temperatur von 145°C rührbar und enthielt 72 % waschaktive Substanz (Epton-Titration) und einen Hydroxiethansulfonat-Gehalt von 3,9 %.

### Beispiel 2

In einem 2 l Planschliffbecher mit Rührer, absteigender Destillationsbrücke, Innenthermometer und Stickstoffeinleitung wurden 230 g Cocosfettsäure, 242 g wäßrige Natriumisethionat-Lösung (58 %) und 8,8 g wäßrige Isethionsäure-Lösung (85 %) vorgelegt. Der Ansatz wurde bei 130°C unter Abdestillieren des bei der Reaktion gebildeten Wasser bis zu einem Gehalt an waschaktiver Substanz von 79 % kondensiert und die derart erhaltene Schmelze wurde mit 16,4 g Ammoniumstearat versetzt. Das so erhaltene Natrium-/Ammoniumcocoylisethionat-Gemisch war noch bei einer Temperatur von 128°C rührbar und enthielt 85 % waschaktive Substanz (Epton-Titration) sowie 3,5 % freies Hydroxiethansulfonat.

### Beispiel 3

In einem 2 l Planschliffbecher mit Rührer, absteigender Destillationsbrücke, Innenthermometer und Stickstoffeinleitung wurden 230 g Cocosfettsäure, 243 g wäßrige Natriumisethionat-Lösung (58 %) und 9,0 g wäßrige Isethionsäure-Lösung (85 %) vorgelegt und der Ansatz bei 130°C unter Abdestillieren des bei der Reaktion gebildeten Wasser kondensiert. Bei einem Gehalt an waschaktiver Substanz von 83 % wurde 1 Liter Ammoniakgas zugegeben und durchgerührt. Die derart erhaltene Natrium-/Ammoniumcocoylisethionat-Schmelze war bei einem WAS-Gehalt (Epton-Titration) von 80 % und einem Gehalt an freiem Hydroxiethansulfonat von 4,6 % noch bei einer Temperatur von 110°C rührbar. Nach Zusatz von 62 g Stearinsäure betrug der WAS-Gehalt 71 %, der Gehalt an freiem Hydroxiethansulfonat 3,9 %, die Rührbarkeitsgrenze lag bei 85°C.

### Beispiel 4

In Analogie zu Beispiel 4 wurden 230 g Cocosfettsäure, 244 g wäßrige Natriumisethionat-Lösung (58 %) und 9,1 g wäßrige Isethionsäure-Lösung (85 %) bei 130°C kondensiert. Bei einem WAS-Gehalt von 81 % wurden 5,14 g Triethylamin zugegeben und gut durchgerührt. Die Schmelze war bei einem WAS-Gehalt (Epton-Titration) von 78 % und einem Gehalt an freiem Hydroxiethansulfonat von 4,8 % noch bei einer Temperatur von 128°C rührbar. Nach Zusatz von 62 g Stearinsäure betrug der WAS-Gehalt 69 %, der Hydroxiethansulfonat-Gehalt 4,0 %, die Rührbarkeitsgrenze lag bei 65°C.

## Patentansprüche

1. Tensidmischungen mit einem Gehalt an Acyloxialkansulfonaten, hergestellt durch Umsetzung einer oder mehrerer Fettsäuren mit (a) einem Alkali- und/oder Erdalkalihydroxialkansulfonat oder (b) einem Gemisch aus Alkali- und/oder Erdalkalihydroxialkansulfonat und Hydroxialkansulfonsäure, wobei vor, während oder nach Beendigung der Reaktion im Fall (a) ein Ammoniumsalz und im Fall (b) ein Ammoniumsalz und/oder ein Amin zugegeben wird.

2. Tensidmischungen nach Anspruch 1, dadurch gekennzeichnet, daß das Acyloxialkansulfonat ein Acylisethionat ist.

3. Tensidmischungen nach Anspruch 1, dadurch gekennzeichnet, daß das Alkali- und/oder Erdalkalihydroxialkansulfonat ein Natrium- oder Kaliumhydroxialkansulfonat ist.

4. Tensidmischungen nach Anspruch 1, hergestellt durch Umsetzung mit Cocosfettsäuren.

5. Tensidmischungen nach Anspruch 1, dadurch gekennzeichnet, daß das Ammoniumsalz Ammoniumstearat ist.

6. Tensidmischungen nach Anspruch 1, dadurch gekennzeichnet, daß das Acyloxialkansulfonat als gemischtes Natrium- und Ammoniumsalz vorliegt mit einem molaren Verhältnis von Na zu NH₄ von 98:2 bis 5:95.
